(19) Europäisches Patentamt
European Patent Office
Office européen des brevets

(11) Veröffentlichungsnummer : **0 199 951 B1**

(12) # EUROPÄISCHE PATENTSCHRIFT

(45) Veröffentlichungstag der Patentschrift : **23.01.91 Patentblatt 91/04**

(51) Int. Cl.[5] : **C07D 213/74,** C07D 213/75

(21) Anmeldenummer : **86103517.8**

(22) Anmeldetag : **15.03.86**

(54) **Verfahren zur Herstellung von 2-Amino-3-nitro-6-(4-fluor-benzyl-amino)-pyridin sowie 2-Amino-3-carbethoxyamino-6-(4-fluor-benzyl-amino)-pyridin.**

Die Akte enthält technische Angaben, die nach dem Eingang der Anmeldung eingereicht wurden und die nicht in dieser Patentschrift enthalten sind.

(30) Priorität : **23.03.85 DE 3510623**

(43) Veröffentlichungstag der Anmeldung : **10.12.86 Patentblatt 86/45**

(45) Bekanntmachung des Hinweises auf die Patenterteilung : **23.01.91 Patentblatt 91/04**

(84) Benannte Vertragsstaaten : **AT BE CH DE FR GB IT LI LU NL SE**

(56) Entgegenhaltungen :
**EP-A- 0 110 091**
**BE-A- 764 362**
**GB-A- 1 191 302**

(73) Patentinhaber : **ASTA Pharma Aktiengesellschaft**
**Weismüllerstrasse 45**
**D-6000 Frankfurt am Main 1 (DE)**

(72) Erfinder : **Orth, Winfried, Dr.**
**Am Schachfeldgraben 28**
**D-6735 Hassloch (DE)**
Erfinder : **Engel, Jürgen, Dr.**
**Erlenweg 3**
**D-8755 Alzenau (DE)**
Erfinder : **Emig, Peter, Dr.**
**Taunusstrasse 6**
**D-6369 Niederdorfelden (DE)**
Erfinder : **Scheffler, Gerhard, Dr.**
**Frankenwaldstrasse 19**
**D-6450 Hanau (DE)**
Erfinder : **Pohle, Hans**
**Marienfelder Strasse 23**
**D-4800 Bielefeld 14 (DE)**

**EP 0 199 951 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Aus der deutschen Patentschrift 1 695 637 ist es bekannt, in 3-Nitropyridinen eine 6-ständige Alkoxygruppe gegen eine substituierte Aminogruppe auszutauschen. Diese Reaktion ist jedoch nicht mehr ohne weiteres durchführbar, wenn in der umzusetzenden Pyridinverbindung sich zusätzlich in der 2-Stellung eine Aminogruppe befindet. In einem derartigen Fall erfolgt nämlich zu einem großen Teil auch ein Austausch dieser Aminogruppe.

Es wurde nun überraschend gefunden, daß bei der Umsetzung von 2-Amino-3-nitro-6-methoxy-pyridin mit 4-Fluor-benzylamin in Wasser als Reaktionsmedium ausschließlich ein Austausch der 6-ständigen Methoxygruppe gegen das 4-Fluor-benzylamin erfolgt, das heißt das gewünschte Endprodukt in 96%iger Ausbeute erhalten wird. Demgegenüber sind die Ausbeuten in anderen Lösungsmitteln, wie zum Beispiel Alkoholen, Dioxan, Toluol oder Mischungen dieser Mittel mit Wasser ganz erheblich niedriger, wobei nun zugleich auch ein Austausch der 2-ständigen Aminogruppe erfolgt, und man schwer trennbare Gemische erhält, unter denen sich auch stets erhebliche Mengen an nicht umgesetzter Ausgangspyridin-Verbindung befinden.

Das 2-Amino-3-nitro-6-(4-fluor-benzylamino)-pyridin ist ein wichtiges Zwischenprodukt für die Herstellung des analgetisch wirksamen Arzneimittelwirkstoffs 2-Amino-3-carbethoxyamino-6-(4-fluorbenzylamino)-pyridin und stellt einen zweiten und neuen verbesserten Weg für die Herstellung dieses Wirkstoffes dar. Die bisherige Herstellung erfolgte durch Nitrierung von 2,6-Dichlor-pyridin, Umsetzung des so erhaltenen 2,6-Dichlor-3-nitro-pyridins mit Ammoniak unter Austauch des 2-ständigen Chloratoms gegen die Aminogruppe und anschließende Umsetzung mit 4-Fluor-benzylamin, wobei nun das Chloratom in 6-Stellung durch den 4-Fluorbenzylamino-Rest ersetzt wird. Man erhält auf diese Weise dann das 2-Amino-3-nitro-6-(4-fluor-benzylamino)-pyridin (siehe Belgisches Patent 764 362 sowie Europa-Anmeldung 110 091). Das bei diesem Herstellungsweg verwendete 2,6-Dichlor-3-nitro-pyridin hat jedoch den großen Nachteil, daß es eine sehr reaktive Substanz ist, welche Allergien hervorruft ; darüberhinaus ist bereits die Herstellung dieser Substanz (Nitrierung von 2,6-Dichlor-pyridin) nicht ungefährlich indem diese gegebenenfalls explosionsartig verläuft.

In der Britischen Patentschrift 1 191 302 wird zwar in sehr allgemeiner Form eine entsprechende Umsetzung angegeben, jedoch ohne Beispiele sowie ohne konkrete Einzelheiten. Die erfindungsgemäße Umsetzung, die nur in Wasser abläuft, ist daher aus dieser britischen Patentschrift nicht zu entnehmen. Dementsprechend werden die Pyridin-Verbindungen gemäß dieser Britischen Patentschrift stets durch Umsetzung eines Amins mit 2-Amino-3-nitro-6-chlor-pyridin erhalten, wobei letzteres wiederum aus dem 2,6-Dichlor-3-nitro-pyridin hergestellt werden mußte und wo wiederum die oben geschilderten Nachteile vorhanden sind.

Der erfindungsgemäß vorgeschlagene Syntheseweg bedeutet daher eine erhebliche Vereinfachung, größere Sicherheit und verbesserte Umweltfreundlichkeit bei der Herstellung von 2-Amino-3-carbethoxyamino-6-(4-fluor-benzylamino)-pyridin.

Das erfindungsgemäße Verfahren der Umsetzung mit dem 4-Fluor-benzylamin wird in Wasser bei Temperaturen zwischen 70 und 150°C, vorzugsweise zwischen 90 und 120°C durchgeführt. Gegebenenfalls wird bei Temperaturen über 100°C im Autoklaven gearbeitet. Zweckmäßig wird das Verfahren unter Rühren durchgeführt.

Auf 1 Mol 2-Amino-3-nitro-6-methoxy-pyridin werden beispielsweise 1 bis 4 Mol, vorzugsweise 2 Mol 4-Fluor-benzylamin verwendet. Das 4-Fluor-benzylamin kann auch als Salz eingesetzt werden. Insbesondere kommen die Salze des 4-Fluor-benzylamins mit anorganischen Säuren beziehungsweise Mineralsäuren in Frage (zum Beispiel Hydrochlorid, Hydrosulfat, Sulfat). In diesem Falle ist es erforderlich, daß die wässrige Reaktionsmischung zur Freisetzung der 4-Fluor-benzylamin-Base vor oder während des Erwärmens mit der jeweiligen stöchiometrischen Menge einer basischen Substanz (zweckmäßig als wässrige Lösung) versetzt wird. Als basisische Substanzen kommen hierfür beispielsweise in Frage : Alkalihydroxyde (NaOH, KOH), Alkalicarbonate ($K_2CO_3$, $Na_2CO_3$), tertiäre Amine, vorzugsweise niedere alipathische Amine (Triethylamin). Falls das 4-Fluor-benzylamin in Form eines Salzes eingesetzt wird, kann beispielsweise auch wie folgt verfahren werden :

Eine Lösung von 2 Mol des 4-Fluor-benzylaminsalzes in 200-900 ml Wasser, vorzugsweise in 400 ml Wasser, wird mit 2 Mol einer basischen Substanz in beispielsweise 100-200 ml Wasser neutralisiert und diese Mischung dann zu einer Suspension von 1 Mol der Pyridin-Ausgangskomponente in 1-1,7 Liter Wasser, vorzugsweise 1,5 Liter Wasser unter Rühren gegeben und die so erhaltene Mischung erwärmt.

Die Reaktionszeit beträgt in Abhängigkeit von der Reaktionstemperatur zwischen 5 und 15 Stunden. Beispielsweise beträgt die Reaktionszeit bei 75°C 14 Stunden, bei 120°C im Autoklaven 7,5 Stunden.

Pro 1 Mol 2-Amino-3-nitro-6-methoxy-pyridin werden beispielsweise 1 bis 3,5 Liter, vorzugsweise 1,5

bis 2,5 Liter Wasser verwendet.

Zweckmäßig wird die Reaktionsmischung nach Beendigung der Reaktion einer Wasserdampfdestillation bei Normaldruck oder unter vermindertem Druck (200 mbar bis 20 mbar) unterworfen, wobei nicht umgesetztes 4-Fluor-benzylamin azeotrop abdestilliert und aus dem wässrigen Destillat durch Extraktion mit Diethylether zurückgewonnen und als Ausgangsmaterial wieder verwendet werden kann.

Für die Reduktion der Nitrogruppe hat sich als besonders geeignet die katalytische Hydrierung erwiesen. Als Katalysatoren kommen zum Beispeil in Frage :
Raney-Nickel, Edelmetalle wie Palladium und Platin sowie Verbindungen davon, mit und ohne Träger, wie beispielsweise Bariumsulfat, Calciumsulfat und so weiter. Es empfiehlt sich, die Hydrierung der Nitrogruppe bei Temperaturen zwischen 20 und 100°C und einem Druck von ungefähr 1 bis 70 bar in einem Lösungsmittel vorzunehmen. Als Lösungsmittel eignen sich beispielsweise $C_1$-$C_4$-Alkanole, cyclische Ether wie Dioxan, Tetrahydrofuran, Methoxy-ethanol, Wasser, aromatische Kohlenwasserstoffe (Benzol, Toluole, Xylole) sowie Gemische dieser Mittel. Für die anschließende Isolierung der reduzierten Verbindungen kann es in manchen Fällen von Vorteil sein, wenn zu Beginn dem zu hydrierenden Gemisch Trockenmittel, wie wasserfreies Natrium- oder Magnesiumsulfat zugesetzt werden.

Die Reduktion kann aber auch mit nascierendem Wasserstoff beispielsweise Zink/Salzsäure, Zinn/Salzsäure, Eisen/ Salzsäure oder mit Salzen des Schwefelwasserstoffs in Alkohol/Wasser bei etwa 70 bis etwa 120°C oder mit aktiviertem Aluminium in wasserhaltigem Äther bei 20 bis 40°C oder mit Zinn (II)-Chlorid/Salzsäure durchgeführt werden.

Das so erhaltene Reaktionsprodukt wird zweckmäßig sofort in der anfallenden Reaktionsmischung mit einer Verbindung umgesetzt, die geeignet ist, ein Wasserstoffatom der durch die Reduktion erhaltenen 3-ständigen Aminogruppe durch die Carbethoxygruppe $-CO-OC_2H_5$ zu ersetzen, ohne daß das 2,3-Diamino-6-benzylamino-pyridin-derivat isoliert werden muß. Insbesondere gilt dies für den Fall der katalytischen Hydrierung. Selbstverständlich kann diese zuletzt genannte Verbindung auch isoliert werden und dann die Carbethoxygruppe eingeführt werden. Die Einführung kann in der hierfür üblichen Weise mit den hierfür üblichen Reagenzien erfolgen. Beispiele für solche Reagenzien sind : Halogenameisensäureethylester, wie Chlor-, Brom- oder Jodameisensäureethylester. Da das freie Ausgangsamin (2,3-Diamino-pyridin-Derivat) sauerstoffempfindlich ist, wird zweckmäßig unter Stickstoffatmosphäre gearbeitet.

Die Einführung der Carbethoxygruppe wird im allgemeinen in einem inerten Lösungs- oder Suspensionsmittel bei Temperaturen zwischen 0 bis 60°C, insbesondere 5 bis 40°C, vorzugsweise 20 bis 25°C durchgeführt. Als Lösungsmittel kommen beispielsweise in Betracht : Gesättigte alicyclische und cyclische Ether (Dioxan, Tetrahydrofuran, niedere Dialkylether wie Diethylether, Diisopropylether), niedere Alkanole wie Ethanol, Isopropanol, Butanol, niedere alipathische Ketone (Aceton, Methylethylketon), niedere alipathische Kohlenwasserstoffe oder Halogenwasserstoffe (Methylenchlorid, Chloroform, 1,2-Dichlorethan), aromatische Kohlenwasserstoffe (Benzol, Toluol, Xylol), niedere Dialkylamide von niederen gesättigten alipathischen Carbonsäuren (Dimethylformamid, Dimethylacetamid), Tetramethylharnstoff, N-Methylpyrrolidon, Dimethylsulfoxid beziehungsweise Mischungen dieser Mittel.

Im allgemeinen werden die Reaktionskomponenten in molaren Mengen umgesetzt. Gegebenenfalls kann es jedoch zweckmäßig sein, eine Reaktionskomponente in leichtem Überschuß einzusetzen. Gegebenenfalls kann die Umsetzung auch in Gegenwart von basischen beziehungsweise säurebindenden Mitteln wie Alkalicarbonaten (Pottasche, Soda), Alkalihydrogencarbonaten, Alkaliacetaten, Alkalihydroxyden oder tertiären Aminen (beispielsweise Triethylamin) durchgeführt werden. Letzteres gilt insbesondere, wenn Halogenameisensaüreester eingesetzt werden.

Je nach den Verfahrensbedingungen und Ausgangsstoffen erhält man die Verfahrensprodukte in freier Form oder in Form ihrer Salze. Diese Salze können in an sich bekannter Weise, beispielsweise mit Alkali oder Ionenaustauschern, wieder in die freie Base übergeführt werden. Von letzterer lassen sich durch Umsetzung mit organischen oder anorganischen Säuren wieder die Salze gewinnen. Als solche Säuren seien beispielsweise genannt : Halogenwasserstoffsäuren, Schwefelsäure, Phosphorsäuren, Salpetersäure, Perchlorsäure, organische Mono-, Di-oder Tricarbonsäuren der aliphatischen, alicyclischen, aromatischen oder heterocyclischen Reihe sowie Sulfonsäuren. Beispiele hierfür sind : Ameisen-, Essig-Propion-, Bernstein-, Glykol- Milch-, Äpfel-, Wein-, Zitronen-, Ascorbin-, Malein-, Fumar-, Hydroxymalein- oder Brenztraubensäure ; Phenylessig-, Benzoe-, p-Amino-benzoe-, Anthranil-, p-Hydroxy-benzoe-, Salicyl- oder p-Amino-salicylsäure, Embonsäure, Methansulfon-, Äthansulfon-, Hydroxyäthansulfon-, Äthylensulfonsäure ; Halogenbenzolsulfon-, Toluolsulfon-, Naphthalinsulfonsäure oder Sulfanilsäure.

## Beispiel

Ein Gemisch von 33,8 g (0,2 Mol) 2-Amino-3-nitro-6-methoxy-pyridin und 50,1 g (0,4 Mol) 4-Fluor-benzylamin in 400 ml Wasser wurde 10 Stunden am Rückflusskühler zum Sieden erhitzt, sodann am absteigenden Kühler im Verlauf von 3 Stunden ein Gemisch von Wasser und 4-Fluor-benzylamin abdestilliert[1]. Man kühlte die zurückbleibende Suspension, saugte die kristallin anfallende Verbindung ab, reinigte mit Wasser und trocknete im Vakuum.

Ausbeute : 49,9 g (95,2% der Theorie)
F. 172-176°C (Zersetzung)

Beispiel für die Reduktion der Nitrogruppe und Einführung der Carbethoxygruppe.

2- Amino-3-carbethoxyamino-6-(4-fluor-benzylamino)-pyridin

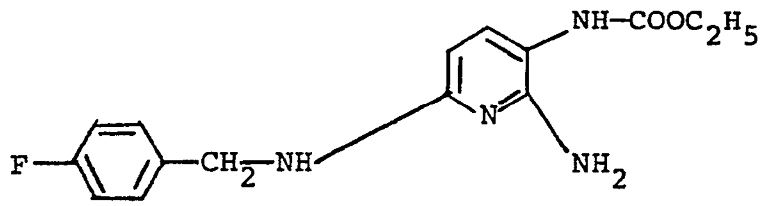

26,2 g (0,1 Mol) 2-Amino-3-nitro-6-(4-fluor-benzylamino)-pyridin werden mit 15 g Raney-Nickel in 250 ml Dioxan bei 50°C und 30 afü (31 bar) hydriert. Die vom Katalysator abgesaugte Lösung wird unter Rühren mit 10,8 ml (0,13 Mol) Chlorameisensäureethylester versetzt, wobei das Hydrochlorid nach ca. 15 Minuten auskristallisiert. Dieses wird abgesaugt und aus $H_2O$ umkristallisiert. Ausbeute : 19 g ;
F. des Hydrochlorids 214-215°C.

[1] Das Destillat wurde mit Ether extrahiert, getrocknet und der Ether im Vakuum abdestilliert.
Rückstand an 4-Fluor-benzylamin : 19 g

## Ansprüche

1. Verfahren zur Herstellung von 2-Amino-3-nitro-6-(4-fluor-benzylamino)-pyridin, dadurch gekennzeichnet, daß man 2-Amino-3-nitro-6-methoxypyridin mit 4-Fluor-benzylamin oder einem Salz hiervon in Wasser bei einer Temperatur zwischen 70 und 150°C umsetzt.

2. Verfahren zur Herstellung von 2-Amino-3-Carbethoxyamino-6-(4-fluor-benzylamino)-pyridin, dadurch gekennzeichnet, daß man
a) 2-Amino-3-nitro-6-(4-fluor-benzylamino)-pyridin gemäß Anspruch 1 herstellt und
b) in der so erhaltenen Verbindung die Nitroguppe zur Aminogruppe reduziert, in diese 3-ständige Aminogruppe durch Acylierung die Carbethoxygruppe einführt und gegebenenfalls die Verfahrensprodukte in ihre Säureadditionssalze überführt.

## Claims

1. Process for the preparation of 2-amino-3-nitro-6-(4-fluoro-benzylamino)-pyridine, characterised in that 2-amino-3-nitro-6-methoxypyridine is reacted with 4-fluoro-benzylamine or a salt thereof in water at a temperature from 70°C to 150°C,

2. Process for the preparation of 2-amino-3-carbethoxyamino-6-(4-fluoro-benzylamino)-pyridine, characterised in that
a) 2-Amino-3-nitro-6-(4-fluoro-benzylamino) -pyridine is prepared according to Claim 1 and
b) the nitro group in the resulting compound is reduced to the amino group, the carbethoxy group is introduced into this 3-position amino group by acylation and the products of the process are optionally converted into their acid addition salts.

## Revendications

1. Procédé de préparation de 2-amino-3-nitro-6-(4-fluorobenzylamino)-pyridine, caractérisé en ce

qu'on fait réagir de la 2-amino-3-nitro-6-méthoxypyridine avec de la 4-fluorobenzylamine ou un sel de celle-ci dans l'eau à une température comprise entre 70 et 150°C.

2. Procédé de préparation de 2-amino-3-carbétnoxyamino-6-(4-fluorobenzylamino)-pyridine, caractérisé en ce que

a) on prépare de la 2-amino-3-nitro-6-(4-fluorobenzylamino)-pyridine selon la revendication 1 et

b) dans le composé ainsi obtenu, on réduit le groupement nitro en groupement amino, on introduit le groupement carbéthoxy dans ce groupement amino en position 3 par acylation et on transforme éventuellement les produits du procédé en leurs sels d'addition d'acides.